# EUROPEAN PATENT APPLICATION

(11) **EP 4 316 412 A2**
(43) Date of publication of application: **07.02.2024**
(21) Application number: 23215622.4
(22) Date of filing: 15.03.2019
(51) Int. Cl.: A61B 90/00

(54) **NAVIGATION SLEEVE FOR MEDICAL INSTRUMENT**

(30) Priority: 16.03.2018 US 201815923152
(62) Divisional of application: 19163211.6
(71) Applicant: Acclarent, Inc., Irvine, CA 92618 (US)
(72) Inventor: SHAMELI, Ehsan, Irvine, 92618 (US); FANG, Itzhak, Irvine, 92618 (US); EBRAHIMI, Babak, Irvine, 92618 (US)
(74) Representative: Carpmaels & Ransford LLP

(57) **Abstract**

An apparatus includes a cylindraceous sleeve body, a navigation sensor, and an interface feature. The cylindraceous sleeve body includes an open proximal end, an open distal end, and a lumen extending from the open proximal end to the open distal end. The lumen is sized and configured to receive a shaft of a medical instrument. The navigation sensor is positioned at the open distal end of the cylindraceous sleeve body. The interface feature is configured to couple the navigation sensor with an image guidance system. The navigation sensor is configured to cooperate with an image guidance system to provide feedback indicating a position of the navigation sensor in three-dimensional space.

## Description

### BACKGROUND

Image-guided surgery (IGS) is a technique where a computer is used to obtain a real-time correlation of the location of an instrument that has been inserted into a patient's body to a set of preoperatively obtained images (e.g., a CT or MRI scan, 3-D map, etc.), such that the computer system may superimpose the current location of the instrument on the preoperatively obtained images. In some IGS procedures, a digital tomographic scan (e.g., CT or MRI, 3-D map, etc.) of the operative field is obtained prior to surgery. A specially programmed computer is then used to convert the digital tomographic scan data into a digital map. During surgery, special instruments having sensors (e.g., electromagnetic coils that emit alternating current electromagnetic fields and/or are responsive to externally generated alternating current electromagnetic fields) mounted thereon are used to perform the procedure while the sensors send data to the computer indicating the current position of each surgical instrument. The computer correlates the data it receives from the instrument-mounted sensors with the digital map that was created from the preoperative tomographic scan. The tomographic scan images are displayed on a video monitor along with an indicator (e.g., crosshairs or an illuminated dot, etc.) showing the real-time position of each surgical instrument relative to the anatomical structures shown in the scan images. In this manner, the surgeon is able to know the precise position of each sensor-equipped instrument by viewing the video monitor even if the surgeon is unable to directly visualize the instrument itself at its current location within the body.

An example of an electromagnetic IGS systems that may be used in ENT and sinus surgery is the CARTO^{®} 3 System by Biosense-Webster, Inc., of Irvine, California. When applied to functional endoscopic sinus surgery (FESS), balloon sinuplasty, and/or other ENT procedures, the use of IGS systems allows the surgeon to achieve more precise movement and positioning of the surgical instruments than can be achieved by viewing through an endoscope alone. As a result, IGS systems may be particularly useful during performance of medical procedures where anatomical landmarks are not present or are difficult to visualize endoscopically.

While several systems and methods have been made and used in medical procedures, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1 depicts a schematic view of an exemplary sinus surgery navigation system being used on a patient seated in an exemplary medical procedure chair;
FIG. 2 depicts a schematic view of a navigation sleeve that may be used with the system of FIG. 1;
FIG. 3 depicts a cross-sectional view of the navigation sleeve of FIG. 2, taken along line 3-3 of FIG. 2;
FIG. 4 depicts a cross-sectional view of an exemplary alternative navigation sleeve;
FIG. 5 depicts a side elevational view of an exemplary medical instrument;
FIG. 6 depicts a side elevational view of the navigation sleeve of FIG. 2 coupled with the medical instrument of FIG. 5;
FIG. 7 depicts a side elevational view of an exemplary alternative navigation sleeve coupled with a distal portion of an exemplary alternative medical instrument;
FIG. 8 depicts a schematic view of another exemplary alternative navigation sleeve; and
FIG. 9 depicts a schematic view of another exemplary alternative navigation sleeve.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

It is further understood that any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Exemplary Image Guided Surgery Navigation System

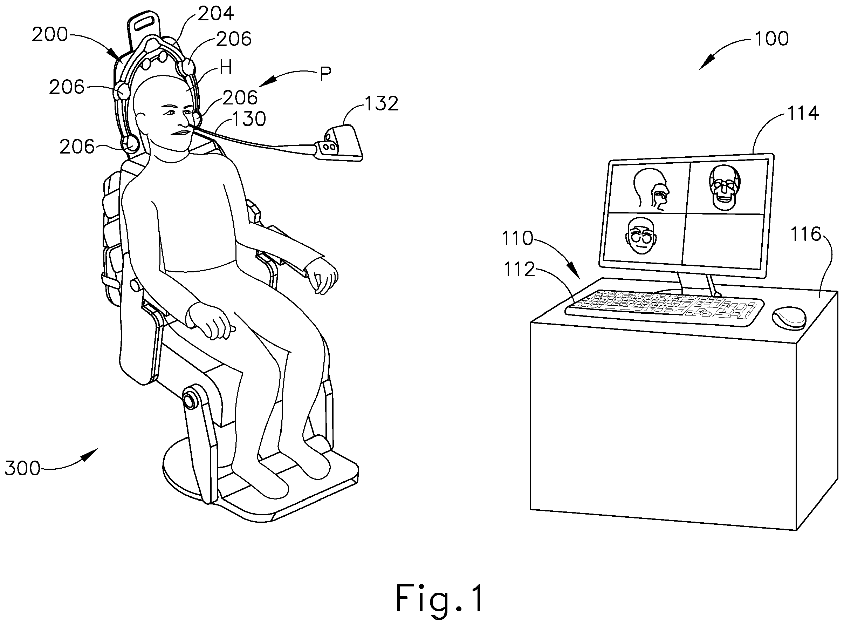

FIG. 1 shows an exemplary IGS navigation system (100) enabling a medical procedure to be performed using image guidance. In some instances, IGS navigation system (100) is used during a procedure where a dilation instrument assembly (not shown) is used to dilate the ostium of a paranasal sinus; or to dilate some other anatomical passageway (e.g., within the ear, nose, or throat, etc.). As another merely illustrative example, IGS navigation system (100) may be used during performance of any other kind of medical procedure within a patient's head, including but not limited to within the patient's nasal cavity, paranasal sinuses, Eustachian tubes, etc.; elsewhere within a patient's head; within a patient's throat; or elsewhere within a patient's body. Various suitable locations and clinical contexts in which IGS navigation system (100) may be used will be apparent to those of ordinary skill in the art in view of the teachings herein.

In addition to or in lieu of having the components and operability described herein IGS navigation system (100) may be constructed and operable in accordance with at least some of the teachings of U.S. Pat. No. 8,702,626, entitled "Guidewires for Performing Image Guided Procedures," issued April 22, 2014, the disclosure of which is incorporated by reference herein; U.S. Pat. No. 8,320,711, entitled "Anatomical Modeling from a 3-D Image and a Surface Mapping," issued November 27, 2012, the disclosure of which is incorporated by reference herein; U.S. Pat. No. 7,720,521, entitled "Methods and Devices for Performing Procedures within the Ear, Nose, Throat and Paranasal Sinuses," issued May 18, 2010, the disclosure of which is incorporated by reference herein; U.S. Pat. Pub. No. 2014/0364725, entitled "Systems and Methods for Performing Image Guided Procedures within the Ear, Nose, Throat and Paranasal Sinuses," published December 11, 2014, the disclosure of which is incorporated by reference herein; U.S. Pub. No. 2016/0310042, entitled "System and Method to Map Structures of Nasal Cavity," published October 27, 2016; and U.S. Pat. Pub. No. 2011/0060214, entitled "Systems and Methods for Performing Image Guided Procedures within the Ear, Nose, Throat and Paranasal Sinuses," published March 10, 2011, the disclosure of which is incorporated by reference herein.

IGS navigation system (100) of the present example comprises a field generator assembly (200), which comprises set of magnetic field generators (206) that are integrated into a horseshoe-shaped frame (204). Field generators (206) are operable to generate alternating magnetic fields of different frequencies around the head of the patient. Field generators (206) thereby enable tracking of the position of a navigation guidewire (130) that is inserted into the head of the patient. Various suitable components that may be used to form and drive field generators (206) will be apparent to those of ordinary skill in the art in view of the teachings herein.

In the present example, frame (204) is mounted to a chair (300), with the patient (P) being seated in the chair (300) such that frame (204) is located adjacent to the head (H) of the patient (P). By way of example only, chair (300) and/or field generator assembly (200) may be configured and operable in accordance with at least some of the teachings of U.S. Patent App. No. 62/555,824, entitled "Apparatus to Secure Field Generating Device to Chair," filed September 8, 2017, the disclosure of which is incorporated by reference herein.

IGS navigation system (100) of the present example further comprises a processor (110), which controls field generators (206) and other elements of IGS navigation system (100). For instance, processor (110) is operable to drive field generators (206) to generate alternating current electromagnetic fields; and process signals from navigation guidewire (130) to determine the location of a sensor (not shown) in navigation guidewire (130) within the head (H) of the patient (P). Processor (110) comprises a processing unit communicating with one or more memories. Processor (110) of the present example is mounted in a console (116), which comprises operating controls (112) that include a keypad and/or a pointing device such as a mouse or trackball. A physician uses operating controls (112) to interact with processor (110) while performing the surgical procedure.

A coupling unit (132) is secured to the proximal end of a navigation guidewire (130). Coupling unit (132) of this example is configured to provide wireless communication of data and other signals between console (116) and navigation guidewire (130). While coupling unit (132) of the present example couples with console (116) wirelessly, some other versions may provide wired coupling between coupling unit (132) and console (116). Various other suitable features and functionality that may be incorporated into coupling unit (132) will be apparent to those of ordinary skill in the art in view of the teachings herein.

Navigation guidewire (130) of the present example includes a sensor (not shown) that is responsive within the fields generated by field generators (206). In the present example, the sensor of navigation guidewire (130) comprises at least one coil at the distal end of navigation guidewire (130). When such a coil is positioned within an alternating current electromagnetic field generated by field generators (206), such positioning may generate electrical current in the coil, and this electrical current may be communicated along the electrical conduit(s) in navigation guidewire (130) and further to processor (110) via coupling unit (132). This phenomenon may enable IGS navigation system (100) to determine the location of the distal end of navigation guidewire (130) within a three-dimensional space (i.e., within the head (H) of the patient (P)). To accomplish this, processor (110) executes an algorithm to calculate location coordinates of the distal end of navigation guidewire (130) from the position related signals of the coil(s) in navigation guidewire (130).

Processor (110) uses software stored in a memory of processor (110) to calibrate and operate system (100). Such operation includes driving field generators (206), processing data from navigation guidewire (130), processing data from operating controls (112), and driving display screen (114). Processor (110) is further operable to provide video in real time via display screen (114), showing the position of the distal end of navigation guidewire (130) in relation to a video camera image of the patient's head (H), a CT scan image of the patient's head (H), and/or a computer generated three-dimensional model of the anatomy within and adjacent to the patient's nasal cavity. Display screen (114) may display such images simultaneously and/or superimposed on each other during the surgical procedure. Such displayed images may also include graphical representations of instruments that are inserted in the patient's head (H), such as navigation guidewire (130), such that the operator may view the virtual rendering of the instrument at its actual location in real time. By way of example only, display screen (114) may provide images in accordance with at least some of the teachings of U.S. Pub. No. 2016/0008083, entitled "Guidewire Navigation for Sinuplasty," published January 14, 2016, the disclosure of which is incorporated by reference herein. In the event that the operator is also using an endoscope, the endoscopic image may also be provided on display screen (114).

Those of ordinary skill in the art will recognize that, when navigation guidewire (130) is coupled with a medical instrument, the images provided through display screen (114) may help guide the operator in maneuvering and otherwise manipulating the medical instrument within the patient's head (H) and/or elsewhere within the anatomy of the patient (P).

### II. Exemplary Navigation Sleeve

Navigation guidewire (130) may be particularly useful with medical instruments (e.g., catheters, etc.) that include a lumen that is capable of accommodating navigation guidewire (130). If navigation guidewire (130) is disposed in such a lumen, and the distance between a sensor in navigation guidewire (130) and a structural feature of the medical instrument is known (e.g., substantially coincident), then IGS system (100) may essentially "know" where that structural feature of the medical instrument is located within the patient (P), based on the sensed location of the sensor in navigation guidewire (130). However, a variety of conventional medical instruments do not include a lumen or other structural feature that would be configured to accommodate navigation guidewire (130). It may therefore be desirable to provide a device that can provide the position-sensing capabilities of navigation guidewire (130), yet be coupled with a conventional medical instrument that lacks a lumen or other structural feature that would be configured to accommodate navigation guidewire (130). The following examples relate to various devices that may be coupled with conventional medical instruments that lack a lumen or other structural feature that would be configured to accommodate navigation guidewire (130), while providing the position-sensing capabilities of navigation guidewire (130).

### A. Exemplary Navigation Sleeve with Wired Communication

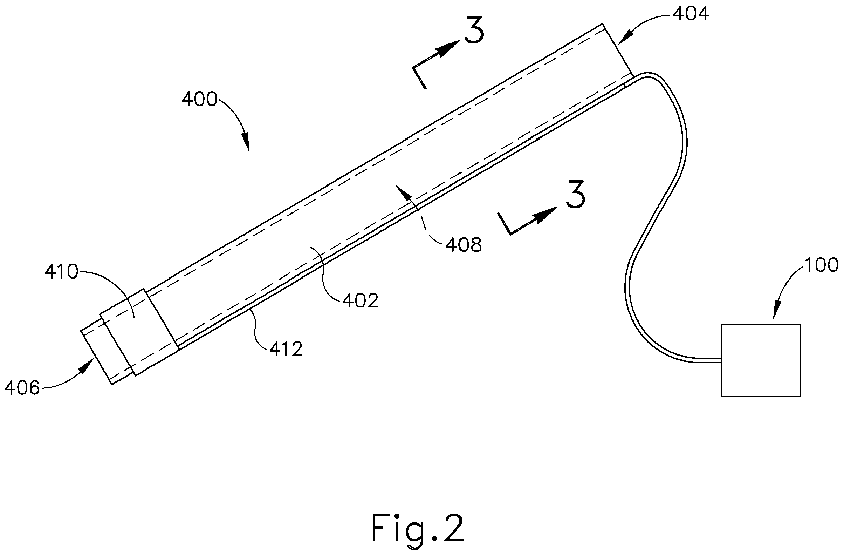

FIGS. 2-3 show an exemplary navigation sleeve (400). Navigation sleeve (400) of this example comprises a substantially cylindraceous body (402) having an open proximal end (404) and an open distal end (406). While body (402) is cylindraceous in the present example, any other suitable configuration (e.g., flat, etc.) may be used. A lumen (408) extends through body (402), from proximal end (404) to distal end (406). Body (402) thus has an inner surface (405) and an outer surface (403). A sensor (410) is secured to outer surface (403) of body (402) near distal end (406). In some versions, an outer tube, sleeve, overmold, shrink wrap, and/or other structure is secured about the exterior of sensor (410), to protect sensor (410) and/or to further secure sensor (410) relative to body (402).

In the present example, sensor (410) comprises a coil wrapped about the longitudinal axis of body (402), thereby forming a cuff-like configuration. While sensor (410) is secured to outer surface (403) of body (402) in the present example, sensor (410) may instead be integrated into the wall of body (402). As yet another merely illustrative alternative, sensor (410) may be positioned against inner surface (405) of body (402). Like the sensor of navigation guidewire (130), sensor (410) of navigation sleeve (400) is configured to generate electrical signals in response to movement of sensor (410) within the alternating current electromagnetic field generated by field generators (206); and processor (110) is configured to process those signals to thereby determine the location of sensor (410) within three-dimensional space. Various suitable configurations and positions that may be used for sensor (410) will be apparent to those of ordinary skill in the art in view of the teachings herein. Some versions may even include two or more sensors (410). In versions with more than one sensor (410), the sensors (410) can be located at the same distance from the distal end; or as an alternative, they might be located with some distance from each other. Using more than one sensor (410) may provide additional information about the navigated instrument, such as certain instrument orientations, or extra confirmation of the location.

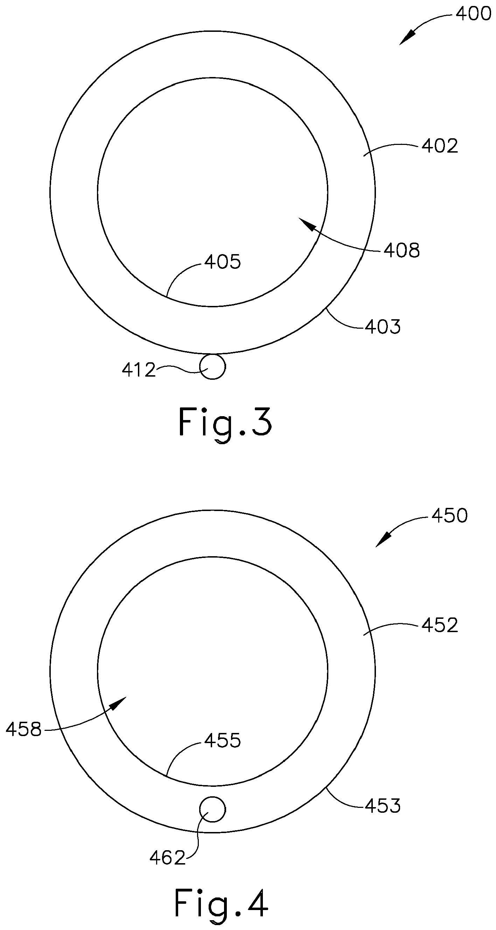

A wire (412) couples sensor (410) with IGS system (100), thereby communicating electrical signals from sensor (410) to IGS system (100). As shown in FIG. 3, wire (412) is positioned along outer surface (403) of body (402). By way of example only, wire (412) may be adhered to outer surface (403) via an adhesive; may be secured to outer surface (403) by one or more bands, straps, cuffs, or the like; may be secured to outer surface (403) by an outer shrink wrap; may be fitted in a longitudinally extending channel or recess formed in outer surface (403); or may be otherwise secured to outer surface (403). Other suitable ways in which wire (412) may be secured to outer surface (403) will be apparent to those of ordinary skill in the art in view of the teachings herein.

FIG. 4 shows another exemplary navigation sleeve (450). Navigation sleeve (450) of this example is substantially the same as navigation sleeve (400) identified above. For instance, navigation sleeve (450) of this example includes a cylindraceous body (452) defining a lumen (458), with an inner surface (455) and an outer surface (453). While body (452) is cylindraceous in this example, any other suitable configuration (e.g., flat, etc.) may be used. Navigation sleeve (450) of this example also includes a wire (462) that couples a sensor (not shown) of navigation sleeve (450) with IGS system (100). Unlike wire (412), wire (462) of this example is embedded within body (452), between surfaces (453, 455). Various suitable ways in which wire (462) may be integrated into body (452) will be apparent to those of ordinary skill in the art in view of the teachings herein.

As yet another merely illustrative alternative, wire (412, 462) may be secured to inner surface (405, 455). By way of example only, wire (412, 462) may be fitted in a longitudinally extending channel or recess formed in inner surface (405, 455); or may be otherwise secured to inner surface (405, 455). Other suitable ways in which wire (412, 462) may be secured to inner surface (405, 455) will be apparent to those of ordinary skill in the art in view of the teachings herein.

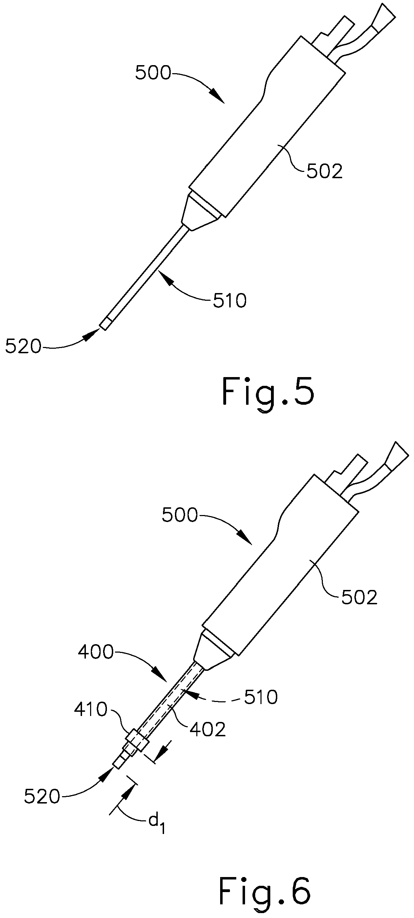

FIG. 5 shows an exemplary medical instrument (500) that comprises a handle assembly (502), a shaft assembly (510) extending distally from handle assembly (502), and an end effector (520) at the distal end of shaft assembly (510). In the present example, shaft assembly (510) is rigid and straight. End effector (520) is operable to engage tissue and modify the tissue, provide therapy to the tissue, and/or otherwise engage the tissue. By way of example only, end effector (520) may comprise a microdebrider, a tissue/bone shaver, and/or any other suitable kind of feature(s). Other suitable forms that end effector (520) may take will be apparent to those of ordinary skill in the art in view of the teachings herein. Medical instrument (500) may be configured for use in sinus surgery, nasal surgery, laryngeal surgery, and/or other kinds of medical procedures.

Medical instrument (500) of the present example lacks a lumen or other feature that would be configured to accommodate navigation guidewire (130). However, navigation sleeve (400) is configured to couple with medical instrument (500), as shown in FIG. 6, to provide position-sensing capabilities like navigation guidewire (130). As shown, lumen (408) is configured to accommodate shaft assembly (510), such that body (402) may be slid over shaft assembly (510). With body (402) slid over shaft assembly (410), sensor (410) is positioned proximal to end effector (520) by a fixed distance (di). By positioning sensor (410) proximal to end effector (520) navigation sleeve (400) does not interfere with normal operation of medical instrument (500). With the fixed distance (di) between sensor (410) and the distal end of end effector (520) being known, processor (110) may determine the position of the distal end of end effector (520) in three-dimensional space based on the sensed position of sensor (410) in three-dimensional space. Processor (110) may thus drive display screen (114) to visually indicate the position of end effector (520) in relation to a video camera image of the patient's head (H), a CT scan image of the patient's head (H), and/or a computer generated three-dimensional model of the anatomy within the patient's head (H). Of course, if end effector (520) is positioned elsewhere within the patient (P) (i.e., in some anatomical region outside of the head (H)), processor (110) may drive display screen (114) to visually indicate the position of end effector (520) in relation to one or more images or models of that other anatomical region.

By way of example only, the operator may use operating controls (112) to input the value of fixed distance (di) in IGS system (100). As another merely illustrative alternative, distance (d1) can be also measured and calibrated via an electromagnetic calibration system that is customized for that specific tool, and that has its own dedicated coil as source of magnetic field. As yet another merely illustrative alternative, an embedded feature can be integrated into field generator assembly (200) so that the surgical instrument can be placed in a controlled location and orientation relative to the field generator assembly (200). In this way, location of sensor (410) may be accurately estimated through measuring the induced current in sensor (410). Other suitable ways in which processor (110) may be informed of fixed distance (di) will be apparent to those of ordinary skill in the art in view of the teachings herein.

While wire (412) is omitted from FIG. 6, wire (412) may simply extend from proximal end (404) to IGS system (100), such that wire (412) is free relative to handle assembly (502). In some such versions, the operator may nevertheless grasp wire (412) with the same hand that grasps handle assembly (502). As another merely illustrative example, wire (412) may be removably secured to handle assembly (502) using one or more straps, clips, and/or other components/techniques as will be apparent to those of ordinary skill in the art in view of the teachings herein.

In order to maintain fixed distance (di), navigation sleeve (400) may be secured to medical instrument (500) in various different ways. By way of example only, proximal end (404) may be secured to handle assembly (502) and/or shaft assembly (510) via adhesive, clips, clasps, snap-fittings, elastomeric fittings, and/or using any other suitable components/techniques as will be apparent to those of ordinary skill in the art in view of the teachings herein. As another merely illustrative example, body (402) may include an elastomeric material forming inner surface (405), such that inner surface (405) may deform to accommodate the outer diameter of shaft assembly (510) and grip shaft assembly (510) through friction. Similarly, the entirety of body (402) may be elastic, with body (402) being resiliently biased to assume a radially contracted state, such that body (402) may expand radially outwardly to accommodate the outer diameter of shaft assembly (510) and grip shaft assembly (510) through friction. As yet another merely illustrative example, navigation sleeve (400) may be configured to shrink against, and thereby adhere to, the outer diameter of shaft assembly (510). Such versions of navigation sleeve (400) may be configured to shrink radially inwardly in response to heat, moisture, an applied voltage, and/or other conditions. Some techniques and features that are used to secure navigation sleeve (400) to medical instrument (500) may require destruction of navigation sleeve (400) to remove navigation sleeve (400) from medical instrument (500). Still other suitable ways in which navigation sleeve (400) may be secured to medical instrument (500) will be apparent to those of ordinary skill in the art in view of the teachings herein.

In some scenarios, after navigation sleeve (400) and medical instrument (500) are used in a medical procedure, it may be desirable to discard navigation sleeve (400) and process medical instrument (500) for use in a subsequent medical procedure. To accomplish this, the operator may simply reverse the action that was used to secure navigation sleeve (400) to medical instrument (500). In scenarios where an adhesive was used to secure navigation sleeve (400) to medical instrument (500), the operator may heat the combination of navigation sleeve (400) and medical instrument (500) to cause the adhesive to release. Other suitable ways in which navigation sleeve (400) may be decoupled from medical instrument (500) will be apparent to those of ordinary skill in the art in view of the teachings herein. In some other scenarios, navigation sleeve (400) and medical instrument (500) may simply be disposed of together after use in a medical procedure. As yet another merely illustrative example, navigation sleeve (400) may be configured such that navigation sleeve (400) may be processed for use in a second medical procedure after navigation sleeve (400) has been used in a first medical procedure.

It may be desirable to enable navigation sleeve (400) to be coupled with various different kinds of medical instruments (500), which may have various lengths of shaft assemblies (510). In some such scenarios, the length of shaft assembly (510) may be less than the length of body (402). To accommodate such shorter shaft assemblies (510), body (402) may be formed of a material enabling body (402) to be easily cut using a conventional knife, shears, or other cutting instrument. In some such versions, body (402) may include a set of indicia spaced apart along the length of body (402) to assist the operator in selecting an appropriate length for body (402) and in cutting body (402) at the appropriate length. As another merely illustrative example, body (402) may include frangible segments, such that the operator may break away one or more segments of body (402) to achieve a desired length for body (402). Such frangible segments may be defined by longitudinally spaced apart annular perforations or other kinds of longitudinally spaced apart weakened annular regions. As yet another merely illustrative example, body (402) may be configured such that body (402) is longitudinally deformable, such that the operator may simply crumple or otherwise longitudinally deform body (402) in order to accommodate a relatively short shaft assembly (510). As still another merely illustrative example, the operator may be presented with a kit containing various navigation sleeves (400) with bodies (402) of different lengths, such that the operator may simply choose the sleeve (400) from the set of sleeves (400) with the best fit length of body (402). Other suitable ways in which navigation sleeves (400) may accommodate shaft assemblies (510) of different lengths will be apparent to those of ordinary skill in the art in view of the teachings herein.

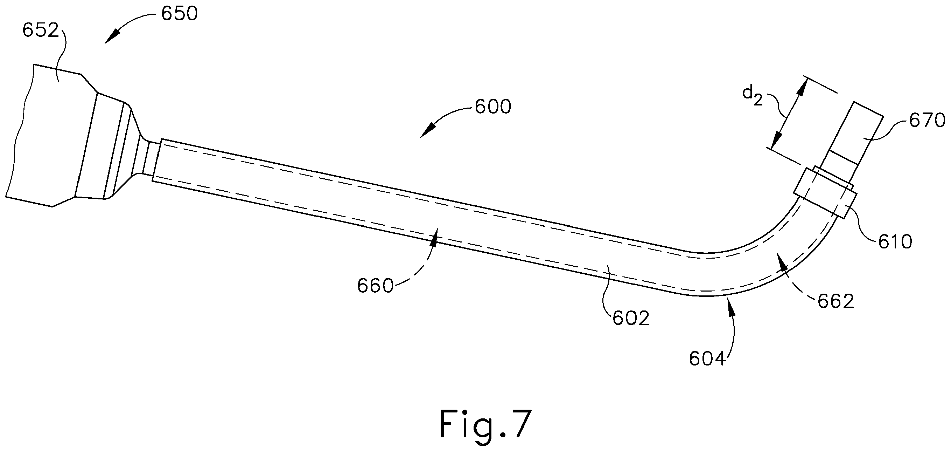

As noted above, some versions of body (402) may be radially deformable and/or longitudinally deformable. In addition, or in the alternative, body (402) may be laterally deformable. FIG. 7 shows an exemplary navigation sleeve (600) with a body (602) that is laterally deformable. Navigation sleeve (600) is secured to a medical instrument (650) that includes a handle assembly (652), a shaft assembly (660), and an end effector (670). A bend (662) is formed in shaft assembly (660), just proximal to end effector (670). As noted above, end effector (670) may comprise a microdebriding feature and/or any other suitable kind(s) of end effector feature(s). With body (602) being laterally deformable, body (602) also defines a curved region (604) along bend (662). A sensor (610) of navigation sleeve (600) is positioned just distal to curved region (604). Sensor (610) may be constructed and operable just like sensor (410) described above. A wire (not shown) may also couple sensor (610) with IGS system (100), just like wire (412) described above.

Sensor (610) is positioned at a fixed distance (d₂) from the distal end of end effector (670). Thus, position data from sensor (610) will be indicative of the position of the distal end of end effector (670) in three-dimensional space. Processor (110) may thus drive display screen (114) to visually indicate the position of end effector (670) in relation to a video camera image of the patient's head (H), a CT scan image of the patient's head (H), and/or a computer generated three-dimensional model of the anatomy within the patient's head (H). Of course, if end effector (670) is positioned elsewhere within the patient (P) (i.e., in some anatomical region outside of the head (H)), processor (110) may drive display screen (114) to visually indicate the position of end effector (670) in relation to one or more images or models of that other anatomical region.

### B. Exemplary Navigation Sleeve with Wireless Communication

As noted above, a wire (412, 462) may be used to directly couple sensor (410) with IGS navigation system (100). As also noted above, this may require accommodation of wire (412, 462) along the length of body (402, 452), which may ultimately result in an expanded effective outer diameter for navigation sleeve (400, 450, 600). It may therefore be desirable to provide a version of navigation sleeve (400, 450, 600) that provides at least some degree of wireless communication, such that it is not necessary to accommodate wire (412, 462) along the length of body (402, 452).

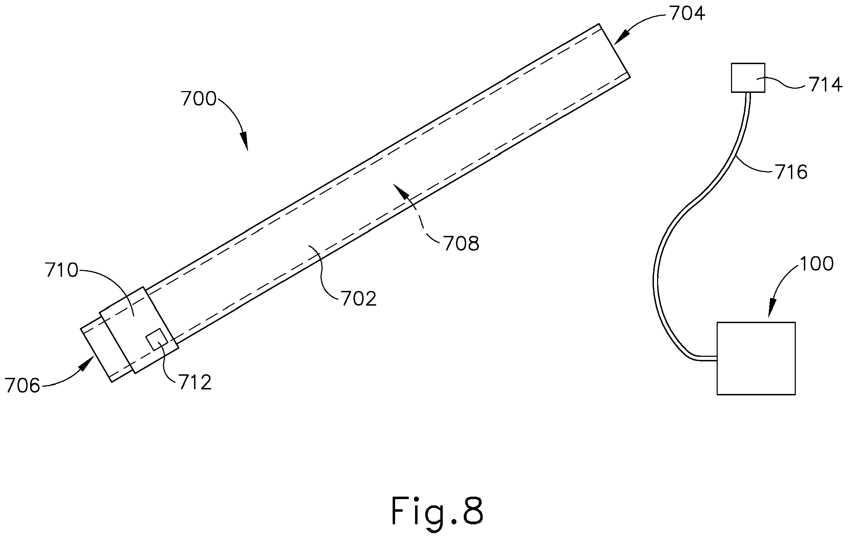

FIG. 8 shows an exemplary alternative navigation sleeve (700) that is substantially similar to navigation sleeve (400). Navigation sleeve (700) of this example comprises a substantially cylindraceous body (702) having an open proximal end (704) and an open distal end (706). While body (702) is cylindraceous in this example, any other suitable configuration (e.g., flat, etc.) may be used. A lumen (708) extends through body (702), from proximal end (704) to distal end (706). A sensor (710) is secured to body (402) near distal end (706). In some versions, an outer tube, sleeve, overmold, shrink wrap, and/or other structure is secured about the exterior of sensor (710), to protect sensor (710) and/or to further secure sensor (710) relative to body (702). Sensor (710) may be configured and operable just like sensor (410) described above, such that sensor (710) is configured to generate electrical signals in response to movement of sensor (710) within the alternating current electromagnetic field generated by field generators (206); and processor (110) is configured to process those signals to thereby determine the location of sensor (710) within three-dimensional space.

Unlike navigation sleeve (400), navigation sleeve (700) of the present example comprises a wireless transmitter module (712) that is in communication with sensor (712). Wireless transmitter module (712) is configured to wirelessly communicate with wireless receiver module (714). Wireless receiver module (714) is coupled with IGS system (100) via a wire (716). In some other versions, wireless receiver module (714) is in wireless communication with IGS system (100), such that wire (716) is omitted. In still other versions, wireless transmitter module (712) may be in direct wireless communication with IGS system (100), such that wireless receiver module (714) and wire (716) are both omitted.

In some versions, wireless receiver module (714) is configured to be mounted to proximal end (704) of body (702). In some other versions, wireless receiver module (714) is configured to be mounted to the patient's face, elsewhere on the patient's head, or elsewhere on the patient's body. As yet another merely illustrative example, wireless receiver module (714) may be configured to be mounted to a chair (300) supporting the patient (P), a bed supporting the patient (P), or some other fixture supporting at least a portion of the patient (P). As still another merely illustrative example, wireless receiver module (714) may be configured to be secured to the physician, such as the physician's wrist, garment, etc. Other suitable places in which wireless receiver module (714) may be located will be apparent to those of ordinary skill in the art in view of the teachings herein.

Wireless transmitter module (712) may include one or more coils, antennae, and/or other features that are configured to provide wireless communication with one or more complementary coils, antennae, and/or other features of wireless receiver module (714). In versions where wireless transmitter module (712) needs electrical power to transmit signals wirelessly to wireless receiver module (714), such power may be provided by a battery in wireless transmitter module (712). Alternatively, wireless transmitter module (712) may be powered inductively, by a feature of wireless receiver module (714), by field generators (206), and/or otherwise. Other suitable ways in which wireless transmitter module (712) may wirelessly communicate signals from sensor (710) to wireless receiver module (714) will be apparent to those of ordinary skill in the art in view of the teachings herein.

With modules (712, 714) wirelessly coupled together, navigation sleeve (700) may be operable just like navigation sleeve (400, 450, 600). Thus, IGS system (100) may determine the location of an end effector (520, 670) on a shaft assembly (510, 660) to which navigation sleeve (700) is coupled, and provide corresponding visual feedback to the operator via display screen (114).

### C. Exemplary Navigation Sleeve with Eddy Current Based Wireless Sensor

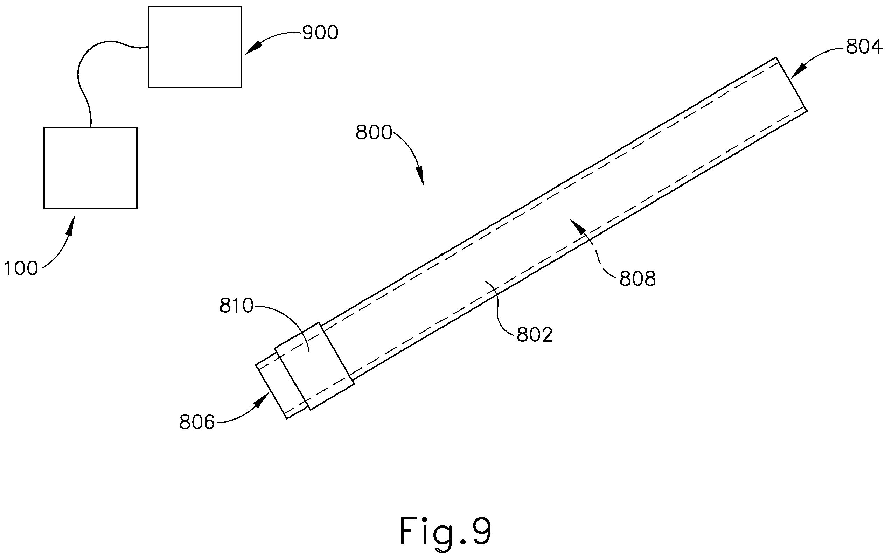

As noted above, it may be desirable to provide a navigation sleeve that does not require the use of any wires extending along the length of sleeve. While navigation sleeve (700) provides one option for accomplishing this goal, FIG. 9 shows another exemplary navigation sleeve (800) that provides yet another merely exemplary option for accomplishing this goal. Navigation sleeve (800) of this example comprises a substantially cylindraceous body (802) having an open proximal end (804) and an open distal end (806). While body (802) is cylindraceous in this example, any other suitable configuration (e.g., flat, etc.) may be used. A lumen (808) extends through body (802), from proximal end (804) to distal end (806). A metallic cuff (810) is secured to body (802) near distal end (806). By way of example only, metallic cuff (810) may comprise aluminum and/or some other electrically conductive material that is capable of generating eddy currents and thereby acts as a noise source within an alternating alternating current electromagnetic field.

Navigation sleeve (800) is configured to be used in conjunction with a coil set (900), which is in communication with IGS system (100). Coil set (900) of this example comprises a plurality of coils (e.g., three coils) that are configured to generate an alternating current electromagnetic field, similar to field generators (206). In the examples described above, field generators (206) only generate alternating current electromagnetic fields, without providing any kind of feedback functionality. However, in the present example, coil set (900) provides feedback functionality in addition to generating an alternating current electromagnetic field. In particular, coil set (900) is operable to act as a receiver, receiving interference or noise feedback signals based on movement of electrically conductive materials in the alternating current electromagnetic field. In the present example, coil set (900) may detect the distance of metallic cuff (810) in multiple axes to locate metallic cuff (810) in three-dimensional space. As metallic cuff (810) gets closer to coil set (900), higher eddy current amplitude (i.e., higher noise) is induced in the conducting material of metallic cuff (810), and this results in less induced current in coil set (900). Coil set (900) is further operable to transmit such noise feedback signals back to IGS system (100). Processor (110) of IGS system (100) is operable to process such feedback signals and thereby determine the location of the interference/noise-inducing object in three-dimensional space.

In the present example, metallic cuff (810) is the electrically conductive object that generates interference or noise within the alternating current electromagnetic field generated by coil set (900). Thus, when the operator moves navigation sleeve (800) within the alternating current electromagnetic field, IGS system (100) may determine the location of metallic cuff (810) within three-dimensional space, based on the interference or eddy current signals generated by metallic cuff (810) as sensed by coil set (900). With navigation sleeve (800) fitted to the shaft of a medical instrument, and with metallic cuff (810) being positioned at a known distance from the distal end of an end effector at a distal end of the shaft of the medical instrument, IGS system (100) may effectively determine the location of the distal end of the end effector of the medical instrument, based on the interference or eddy current signals generated by metallic cuff (810) as sensed by coil set (900).

In some versions, coil set (900) may be placed directly on the face of the patient, to provide greater sensitivity to noise or interference generated by metallic cuff (810) in the alternating current electromagnetic field generated by coil set (900). In addition, the coils of coil set (900) may be wrapped around separate respective coil axes, such that the coil axes are offset from each other. Other suitable ways in which coil set (900) may be configured and positioned will be apparent to those of ordinary skill in the art in view of the teachings herein.

### III. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus, comprising: (a) a cylindraceous sleeve body, wherein the cylindraceous sleeve body includes: (i) an open proximal end, (ii) an open distal end, and (iii) a lumen extending from the open proximal end to the open distal end, wherein the lumen is sized and configured to receive a shaft of a medical instrument; (b) a navigation sensor positioned at the open distal end of the cylindraceous sleeve body; and (c) an interface feature, wherein the interface feature is configured to couple the navigation sensor with an image guidance system, wherein the navigation sensor is configured to cooperate with an image guidance system to provide feedback indicating a position of the navigation sensor in three-dimensional space.

### Example 2

The apparatus of Example 1, wherein the cylindraceous sleeve body is flexible.

### Example 3

The apparatus of any one or more of Examples 1 through 2, wherein the cylindraceous sleeve body is frangible.

### Example 4

The apparatus of Example 3, wherein the cylindraceous sleeve body comprises a longitudinally spaced array of weakened portions configured to facilitate breakage of the body at a corresponding plurality of predetermined longitudinal positions.

### Example 5

The apparatus of any one or more of Examples 1 through 4, wherein the cylindraceous sleeve body is configured to deform laterally.

### Example 6

The apparatus of any one or more of Examples 1 through 5, wherein the cylindraceous sleeve body is configured to deform radially.

### Example 7

The apparatus of any one or more of Examples 1 through 6, wherein the cylindraceous sleeve body is configured to deform longitudinally.

### Example 8

The apparatus of any one or more of Examples 1 through 7, wherein the cylindraceous sleeve body includes an elastomeric material lining the lumen.

### Example 9

The apparatus of any one or more of Examples 1 through 8, wherein the navigation sensor comprises a coil.

### Example 10

The apparatus of Example 9, wherein the cylindraceous sleeve body defines a longitudinal axis, wherein the coil extends around a coil axis that is coaxial with the longitudinal axis of the cylindraceous sleeve body.

### Example 11

The apparatus of any one or more of Examples 1 through 10, wherein the interface feature comprises a wire.

### Example 12

The apparatus of Example 11, wherein the wire extends along an outer surface of the cylindraceous sleeve body.

### Example 13

The apparatus of Example 11, wherein the wire is positioned within a sidewall of the cylindraceous sleeve body.

### Example 14

The apparatus of any one or more of Examples 1 through 13, wherein the interface feature comprises a wireless communication assembly, wherein the wireless communication assembly is configured to provide wireless communication of signals from the navigation sensor to the image guidance system.

### Example 15

The apparatus of Example 14, wherein the wireless communication assembly comprises: (i) a wireless transmitter secured to the cylindraceous sleeve body, (ii) a wireless receiver separated from the cylindraceous sleeve body, and (iii) a wire coupling the wireless receiver with the image guidance system.

### Example 16

The apparatus of any one or more of Examples 1 through 15, further comprising a medical instrument, wherein the medical instrument comprises: (i) a body, (ii) a shaft extending distally from the body, and (iii) an end effector positioned at a distal end of the shaft; wherein the lumen is configured to receive the shaft; wherein the cylindraceous sleeve body has a length such that the end effector may protrude distally from the open distal end of the cylindraceous sleeve body when the shaft is received in the lumen.

### Example 17

The apparatus of Example 16, wherein the shaft has a laterally bent region, wherein the cylindraceous sleeve body has sufficient flexibility to conform to the laterally bent of the shaft.

### Example 18

An apparatus, comprising: (a) a cylindraceous sleeve body, wherein the cylindraceous sleeve body includes: (i) an open proximal end, (ii) an open distal end, and (iii) a lumen extending from the open proximal end to the open distal end, wherein the lumen is sized and configured to receive a shaft of a medical instrument; (b) an eddy current source component positioned at the open distal end of the cylindraceous sleeve body; (c) a plurality of coils forming a coil set, wherein the coil set is configured to generate an alternating current electromagnetic field, wherein the eddy current source is configured to generate noise within the alternating current electromagnetic field, wherein the coil set is further configured to pick up the noise generated by the eddy current source in the alternating current electromagnetic field; and (d) an image guidance system, wherein the image guidance system is configured to drive the coil set to generate the alternating current electromagnetic field, wherein the image guidance system is further configured to receive signals from the coil sets representing noise generated by the eddy current source in the alternating current electromagnetic field, wherein the image guidance system is further configured to determine a position of the eddy current source in three-dimensional space based on signals received from the coil sets representing noise generated by the noise generating component in the alternating current electromagnetic field.

### Example 19

A method of retrofitting a navigation sleeve onto a medical instrument, the method comprising: (a) inserting a shaft of a medical instrument through a lumen of the navigation sleeve; (b) positioning the shaft of the medical instrument relative to the navigation sleeve such that an end effector at a distal end of the shaft is positioned at a fixed distance relative to an open distal end of the navigation sleeve; (c) using the medical instrument in a medical procedure in a patient; (d) using an image guidance system to determine the location of the end effector in the patient, based on signals from a navigation element at the distal end of the navigation sleeve, during use of the medical instrument in the medical procedure.

### Example 20

The method of Example 19, wherein the act of inserting the shaft of the medical instrument through the lumen of the navigation sleeve comprises deforming the navigation sleeve based on a structural configuration of the shaft.

### IV. Miscellaneous

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, versions of the device may be disassembled, and any number of the particular pieces or parts of the device may be selectively replaced or removed in any combination. Upon cleaning and/or replacement of particular parts, versions of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a surgical facility. A device may also be sterilized using any other technique known in the art, including but not limited to beta or gamma radiation, ethylene oxide, or steam.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

### Aspects forming part of the description:

1. An apparatus, comprising:
   (a) a cylindraceous sleeve body, wherein the cylindraceous sleeve body includes:
      (i) an open proximal end,
      (ii) an open distal end, and
      (iii) a lumen extending from the open proximal end to the open distal end, wherein the lumen is sized and configured to receive a shaft of a medical instrument;
   (b) a navigation sensor positioned at the open distal end of the cylindraceous sleeve body; and
   (c) an interface feature, wherein the interface feature is configured to couple the navigation sensor with an image guidance system, wherein the navigation sensor is configured to cooperate with an image guidance system to provide feedback indicating a position of the navigation sensor in three-dimensional space.
2. The apparatus of aspect 1, wherein the cylindraceous sleeve body is flexible.
3. The apparatus of aspect 1, wherein the cylindraceous sleeve body is frangible.
4. The apparatus of aspect 3, wherein the cylindraceous sleeve body comprises a longitudinally spaced array of weakened portions configured to facilitate breakage of the body at a corresponding plurality of predetermined longitudinal positions.
5. The apparatus of aspect 1, wherein the cylindraceous sleeve body is configured to deform laterally, radially or longitudinally.
6. The apparatus of aspect 1, wherein the cylindraceous sleeve body includes an elastomeric material lining the lumen.
7. The apparatus of aspect 1, wherein the navigation sensor comprises a coil.
8. The apparatus of aspect 7, wherein the cylindraceous sleeve body defines a longitudinal axis, wherein the coil extends around a coil axis that is coaxial with the longitudinal axis of the cylindraceous sleeve body.
9. The apparatus of aspect 1, wherein the interface feature comprises a wire.
10. The apparatus of aspect 9, wherein the wire extends along an outer surface of the cylindraceous sleeve body, or is positioned within a sidewall of the cylindraceous sleeve body.
11. The apparatus of aspect 1, wherein the interface feature comprises a wireless communication assembly, wherein the wireless communication assembly is configured to provide wireless communication of signals from the navigation sensor to the image guidance system.
12. The apparatus of aspect 11, wherein the wireless communication assembly comprises:
   (i) a wireless transmitter secured to the cylindraceous sleeve body,
   (ii) a wireless receiver separated from the cylindraceous sleeve body, and
   (iii) a wire coupling the wireless receiver with the image guidance system.
13. The apparatus of aspect 1, further comprising a medical instrument, wherein the medical instrument comprises:
   (i) a body,
   (ii) a shaft extending distally from the body, and
   (iii) an end effector positioned at a distal end of the shaft;

   wherein the lumen is configured to receive the shaft;
   wherein the cylindraceous sleeve body has a length such that the end effector may protrude distally from the open distal end of the cylindraceous sleeve body when the shaft is received in the lumen.
14. The apparatus of aspect 13, wherein the shaft has a laterally bent region, wherein the cylindraceous sleeve body has sufficient flexibility to conform to the laterally bent of the shaft.
15. A method of retrofitting a navigation sleeve onto a medical instrument, the method comprising:
   (a) inserting a shaft of a medical instrument through a lumen of the navigation sleeve;
   (b) positioning the shaft of the medical instrument relative to the navigation sleeve such that an end effector at a distal end of the shaft is positioned at a fixed distance relative to an open distal end of the navigation sleeve;
   (c) using the medical instrument in a medical procedure in a patient;
   (d) using an image guidance system to determine the location of the end effector in the patient, based on signals from a navigation element at the distal end of the navigation sleeve, during use of the medical instrument in the medical procedure.
16. The method of aspect 15, wherein the act of inserting the shaft of the medical instrument through the lumen of the navigation sleeve comprises deforming the navigation sleeve based on a structural configuration of the shaft.

## Claims

1. An apparatus, comprising:
(a) a cylindraceous sleeve body (802), wherein the cylindraceous sleeve body includes:
(i) an open proximal end (804),
(ii) an open distal end (806), and
(iii) a lumen (808) extending from the open proximal end to the open distal end, wherein the lumen is sized and configured to receive a shaft of a medical instrument;
(b) an eddy current source component (810) positioned at the open distal end of the cylindraceous sleeve body;
(c) a plurality of coils forming a coil set (900), wherein the coil set is configured to generate an alternating current electromagnetic field, wherein the eddy current source is configured to generate noise within the alternating current electromagnetic field, wherein the coil set is further configured to pick up the noise generated by the eddy current source in the alternating current electromagnetic field; and
(d) an image guidance system (100), wherein the image guidance system is configured to drive the coil set to generate the alternating current electromagnetic field, wherein the image guidance system is further configured to receive signals from the coil sets representing noise generated by the eddy current source in the alternating current electromagnetic field, wherein the image guidance system is further configured to determine a position of the eddy current source in three-dimensional space based on signals received from the coil sets representing noise generated by the noise generating component in the alternating current electromagnetic field.

2. The apparatus of claim 1, wherein the eddy current source component is a metallic cuff (810) secured to the cylindraceous sleeve body.

3. The apparatus of claim 2, wherein the metallic cuff comprises aluminum.

4. The apparatus of any preceding claim, wherein the coil set comprises three coils.

5. The apparatus of claim 4, wherein the coils of the coil set are wrapped around separate respective coil axes, such that the coil axes are offset from each other.
